(19) Europäisches Patentamt

European Patent Office

Office européen des brevets

(11) **EP 4 123 653 A1**

(12) **EUROPEAN PATENT APPLICATION**

(43) Date of publication:
**25.01.2023 Bulletin 2023/04**

(21) Application number: **21187249.4**

(22) Date of filing: **22.07.2021**

(51) International Patent Classification (IPC):
**G16B 20/10** (2019.01)     **G16B 20/20** (2019.01)

(52) Cooperative Patent Classification (CPC):
**G16B 20/10; G16B 20/20**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **QIAGEN GmbH
40724 Hilden (DE)**

(72) Inventor: **Suryaprakash, Vinay
40721 Hilden (DE)**

(74) Representative: **Vossius & Partner
Patentanwälte Rechtsanwälte mbB
Siebertstraße 3
81675 München (DE)**

(54) **METHOD OF EVALUATING A MUTATIONAL BURDEN**

(57)     The present disclosure relates to a method of evaluating a mutational burden in a sample. The method includes providing first data which represents allelic depths of the sample; calculating rate data based on the first data; segmenting the rate data into a plurality of segments of rate data using a wavelet transform; estimating parameters for each of the plurality of segments; classifying each of the plurality of segments based on the estimated parameters. The classifying includes, for each of a plurality of classification thresholds, generating a classification for the plurality of segments based on the classification threshold and determining an intermediate value based on the classification. The method further comprises determining a mutational burden of the sample based on the intermediate values.

**Fig. 1**

EP 4 123 653 A1

**Description**

The present invention relates to a method of evaluating a mutational burden in a sample and a method of determining a trend of a mutational burden. In the following, the term mutational burden may refer to a tumor mutational burden or tumor cells which are to be identified in a sample.

Examples of the prior art are given in the following. US 9 792 403 B2 relates to the analysis of genetic variants. US 2018/0363066 A1 discloses methods and systems for evaluating tumor mutational burden. Further prior art is given by Sun JX et al. in PLOS Computational Biology, 2018, February, vol. 14, pp. 1-13, "A computational approach to distinguish somatic vs. germline origin of genomic alterations from deep sequencing of cancer specimens without a matched normal". Further prior art is given by Peter Van Loo et al. in Proceedings of the National Academy of Sciences, 2010, vol. 107, no. 39, pp. 16910-16915, "Allele-specific copy number analysis of tumors".

According to the state of the art, however, methods or algorithms are used which can compute the Mutational Burden only in solid tumor samples and require germline controls. They also require coverage depths of 250x or greater. These algorithms also require high purity of 10% or higher and require iterating through the purity values while trying to compute the Mutational Burden. Furthermore, current techniques are not suitable for liquid biopsy samples, or suffer significant losses in accuracy while computing Mutational Burden in samples whose purity is under 10% - which is commonly the case in liquid biopsy samples. Additionally, some of the existing state of the art algorithms also tend to be panel dependent. Due to the limitations listed above, their prognostic efficacy also tends to be low.

These problems are overcome by the present disclosure. The invention comprises a method which solves the problem of finding (or computing) the Mutational Burden (or Load) as well as sample purity for both solid tumor and liquid biopsy samples in a fast and efficient manner for both Whole Exome Sequencing as well as panel-based assays.

The invention is defined in the independent claims. Dependent claims describe preferred embodiments.

In particular, the present disclosure relates to a method of evaluating a mutational burden in a sample. The method includes providing first data which represents allelic depths of the sample; calculating rate data based on the first data; segmenting the rate data into a plurality of segments of rate data using a wavelet transform; estimating parameters for each of the plurality of segments; classifying each of the plurality of segments based on the estimated parameters. The classifying includes, for each of a plurality of classification thresholds, generating a classification for the plurality of segments based on the classification threshold and determining an intermediate value based on the classification. The method further comprises determining a mutational burden of the sample based on the intermediate values.

The mutational burden may be a tumor mutational burden, which can be abbreviated as TMB. The TMB refers to a mutational burden in a tumor. The sample may be a (liquid) biopsy sample. Estimation of the parameters for each of the plurality of segments may be performed based on the first data.

The mutational burden refers to a quantity of mutations in a genome. A tumor mutational burden refers to a quantity of somatic mutations in a genome of a tumor. Usual methods for evaluating a tumor mutational burden require two samples, one of which is a sample from healthy tissue and one of which is a sample from tumor tissue, in order to differentiate between germline mutations and somatic mutations. Germline mutations are mutations of germ cells, which produce gametes such as sperm cells and egg cells. Germline mutations can be passed on to offspring and are then present in every cell in the offspring. Somatic mutations are mutations of somatic cells, also called vegetal cells. Somatic mutations are present only in the descendants of a mutated cell within the same organism. Many cancers are a result of accumulated somatic mutations.

Somatic mutations in the genome of a tumor can allow the immune system of a subject to recognize and fight tumor cells in the body of the subject. In other words, a higher tumor mutational burden indicates that the immune system is in a better position to fight the tumor.

Modern cancer therapies leverage the immune system and improve its capabilities in fighting tumor cells. Such therapies can be called immunotherapies in general. In an immunotherapy, the immune system may be stimulated to more actively fight the tumor cells. Alternatively or additionally, the immunotherapy may support the immune system by reducing or eliminating obstacles which inhibit an immune reaction of the immune system. Alternatively or additionally, the immunotherapy may support the immune system by providing additional immune cells configured to attack tumor cells, for example genetically engineered to attack tumor cells. A higher tumor mutational burden of a patient may be connected with a better responsivity of the patient to an immunotherapy. In other words, the tumor mutational burden is a biomarker that can help providing an improved data basis for therapeutic decisions.

The tumor mutational burden can be a prognostic biomarker, i.e. a biomarker identifying a likelihood of a particular clinical event. A prognostic biomarker is often identified from observational data and used to identify patients more likely to have a particular outcome. If the tumor mutational burden has prognostic capacity, i.e. if it can be used as a prognostic biomarker, it may help identify subjects with a high likelihood of a recurrence. Therefore, a method for evaluating a tumor mutational burden with predictive value may vastly improve therapeutic decisions.

Alternatively or additionally, the tumor mutational burden can be a predictive biomarker, i.e. a biomarker identifying if a subject is more likely to experience a particular effect from exposure to a medical treatment or an environmental agent. A predictive biomarker is generally identified based on a comparison of data of treatment subjects and control subjects with and without the biomarker. Treatment subjects are exposed to the medical treatment or the environmental agent, while control subjects are not exposed to the medical treatment or the environmental agent. If the tumor mutational burden has predictive capacity, i.e. if it can be used as a predictive biomarker, it may help identify subjects more susceptible to an immunotherapy. Therefore, a method for evaluating a tumor mutational burden with predictive value may vastly improve therapeutic decisions.

A sample, also called biopsy sample, refers to a sample extracted from a body of a subject, e.g. from a body of a patient. Methods for evaluating a tumor mutational burden may require a tissue sample from the tumor, i.e. a sample of tumor tissue, to provide useful results. Tissue samples may be obtained from a tumor when the tumor or a part of it are removed in the course of a cancer operation.

More specifically, the sample may be a Formalin-Fixed Paraffin-Embedded sample, also called FFPE sample or FFPE tissue specimen. FFPE is a form of preservation and preparation for biopsy specimens that aids in examination, experimental research, and diagnostic/drug development.

The sample may also be a liquid biopsy sample may refer to a sample of a bodily liquid from a body of a subject. The liquid biopsy sample may be a blood sample, a blood plasma sample, or a urine sample. A tumor in a subject's body may secrete tumor DNA into a bodily liquid, e.g. in the subject's blood or urine. Therefore, a liquid biopsy sample may comprise tumor DNA which circulates freely in the bodily liquid. The purity in a liquid biopsy sample is typically under 10%, often as low as under 1%. However, typical methods for evaluating a tumor mutational burden suffer significant losses in accuracy when applied based on a liquid biopsy sample.

Evaluating a mutational burden based on a liquid biopsy sample is significantly less invasive and unpleasant for a subject compared to evaluating a mutational burden based on a tissue sample. Therefore, a mutational burden may be evaluated more often and tracked more closely, when the mutational burden is evaluated using a liquid biopsy sample. Therefore, a method suitable for evaluating a mutational burden using a liquid biopsy sample may allow for improved diagnostics and treatment of a subject.

The method includes providing first data which represents allelic depths of the sample.

When a sample is analyzed, a read is generated for every position of the genome/exome or panel. A read which does not provide enough statistical evidence to support one allele over another is considered uninformative. The remaining reads supports a particular allele. The allelic depth, also abbreviated AD, is the number of reads that support each of the reported alleles. In other words, the first data represents how often an allele is encountered in a sample.

The first data can, for example, be provided in a General feature format, also called GFF. Such data stores all of the genetic data, much of which is redundant because it will be shared across the genomes.

Preferably, the first data is provided in a Variant Call Format, also called VCF. By using the variant call format only the variations need to be stored along with a reference genome.

In an embodiment, the first data is annotated and the VCF secondary analysis output is obtained. Then, the VCF files for the input sample are parsed. In an embodiment wherein data representing allelic depths of a normal sample are provided as VCF files, the VCF files for the normal sample are parsed along with the VCF files for the input sample.

In an embodiment, the first data comprises multiple smaller VCF files, for example VCF files per chromosome. In this case, the smaller VCF files can be processed in parallel and thus the annotation is performed faster. In this embodiment, the smaller VCF files may be merged into one file after annotation and before parsing.

The method further includes calculating rate data based on the first data. The calculating rate data includes calculating ratios of the allelic depth of the sample to an allelic depth of a normal sample for each position in the genome/exome or panel.

The allelic depth of the normal sample may be an allelic depth of a process-matched normal sample, for example an allelic depth of a sample of healthy tissue obtained using the same process as the allelic depth of the sample. The allelic depths of a process-matched normal sample may be retrieved or generated from a library.

The allelic depth of the normal sample may be an allelic depth of a sample taken at a prior time compared to the sample used in the first data. This way, the method for evaluating a mutational burden requires no healthy sample. Therefore, the method is less invasive and cumbersome for the subject and may be performed even on liquid biopsy samples. For example the sample used for the first data may be a liquid biopsy sample, such as a blood sample, of a subject taken at a later time, and the normal sample may be a liquid biopsy sample, such as a blood sample, of the same subject taken at an earlier time.

The calculating rate data may include calculating a logarithm of the ratio, for example a logarithm to the base 2. The calculating rate data may include parsing the VCF files provided in the previous step of the method.

The method further includes segmenting the rate data into a plurality of segments of rate data using a wavelet transform. The segmenting is a process for dividing the genome/exome into smaller segments of roughly equal copy number. A wavelet transform is applied to the rate data. The wavelet can for example be a linear combination of sinusoids. The resulting transform is processed for splitting the samples into its orthogonal components. Based thereon, a list of breakpoints is generated, which are used as boundaries of the segmentation. Finally, the rate data is segmented according to the list of breakpoints.

The method further includes estimating parameters for each of the plurality of segments. For evaluating the tumor mutational burden, allele frequencies for both somatic and germline mutations per segment are estimated. Further parameters may be estimated or calculated in order to estimate the allele frequencies.

The method further includes classifying each of the plurality of segments based on the estimated parameters. The variants in each of the plurality of segments are classified as either somatic or germline variants. The classifying is carried out using a hypothesis test. The classifying may be carried out using a standard 2-tailed binomial based hypothesis test on the allele frequencies. As a result of the classifying every mutation in each of the segments is classified as somatic or germline.

The classifying includes, for each of a plurality of classification thresholds, generating a classification for the plurality of segments based on the classification threshold and determining an intermediate value based on the classification.

The hypothesis test uses a classification threshold, which can be denominated as $\alpha$. The classification threshold influences the classification. The number of mutations classified as somatic is divided by the length of the respective exon or panel. The result multiplied by 1.000.000 is an intermediate value for the TMB. In prior methods, the classification threshold is selected arbitrarily or merely based on a purity of the sample. The intermediate value determined based on such a classification threshold may not reflect the tumor mutational burden properly.

Multiple intermediate values are determined based on a number of thresholds $\alpha_i$. For example, the intermediate values may be determined iteratively, wherein an intermediate value is determined for a threshold $\alpha_{i+1}$ which is lower than a previous threshold $\alpha_i$, for example $\alpha_{i+1} = \alpha_i/100$.

In one example, more intermediate values are calculated for a sample with lower purity, and fewer intermediate values are calculated for a sample with higher purity.

The method further comprises determining a mutational burden of the sample based on the intermediate values. The mutational burden is determined based on the intermediate values by fitting the intermediate values to a heavy tail distribution, for example to a Pareto distribution. The final estimated mutational burden is may be calculated as the mean of the intermediate values divided by the standard deviation of the intermediate values.

Since the mutational burden is estimated based on a plurality of intermediate values fitted to a heavy tail distribution, the determined mutational burden is more reliable, and the method is more stable. Therefore, the method for evaluating the mutational burden can be used with samples with lower purity and even with liquid biopsy samples.

Various embodiments may preferably implement the following features.

Preferably, the segmenting comprises filtering and fitting the plurality of segments to a distribution.

The filtering may remove outliers from the segmented rate data prior to estimating each of the parameters essential to determining the tumor mutational burden. For example, the segments may be filtered based on the median segment size. In this case, all segments whose length is greater than or equal to one less than the median segment size are retained. In other words, very small segments are discarded.

Preferably, the determining includes fitting the intermediate values to a heavy-tail distribution and calculating one of a mean or a mean scaled by the standard deviation of the fitted heavy-tail distribution; and providing the calculated value as the mutational burden. Even more preferably, the heavy-tail distribution may be a Pareto distribution. This way, the saturating and stabilizing of the intermediate values, which represent TMB value estimates, after a sufficiently low threshold is used for determining a reliable TMB value in little time. Therefore, using the fitting to a heavy tail distribution above, a reliable and robust final estimation for the TMB is obtained very fast.

Preferably, the method further comprises providing second data which represents allelic depths, wherein the rate data is based on the first data and the second data.

The second data represents allelic depths of a normal sample. The normal sample may be for example a process-matched normal sample, as described above. The normal sample may be for example a sample taken at a prior time compared to the sample used in the first data, as described above.

Preferably, the segmenting comprises utilizing a Haar wavelet transform. In this case, the segmenting includes applying a wavelet transform using the Haar wavelet to the rate data.

When the segmenting comprises utilizing a Haar wavelet transform, the segmenting can be especially fast, reliable and parallelizable.

When the rate data comprises logarithms of ratios, the segmenting may be carried out using a Haar Segmentation. In a first sub-step, an undecimated stationary discrete wavelet transform, also called UDTW, using the Haar wavelet is applied to the rate data. In a further sub-step, a set of sub-bands is selected form the transform such obtained. In a further step, the local maxima of the selected sub-bands are calculated. In a further sub-step, a thresholding procedure is applied to the maxima of each sub-band. Preferably, the False Discovery Rate thresholding procedure is applied, where the False Discovery Rate is the proportion of false-positives out of all positives. In a further sub-step, the selected maxima from all the sub-bands are unified. Therein, a list of significant breakpoints in the data is compiled. In a further sub-step, the segmentation result is reconstructed from the list of significant breakpoints. The significant breakpoints are used as boundaries between the segments.

When the segmenting is carried out using a Haar Segmentation, the segmenting can be especially clear, fast and easy to parallelize. Therefore, the segmenting can be particularly well suited for a cloud-based pipeline.

Preferably, the estimated parameters include at least one of purity, ploidy, minor allele copy number, and copy number per segment.

For evaluating the tumor mutational burden, allele frequencies for both somatic and germline mutations per segment are computed based on a number of estimated parameters, namely a purity '$p$', a ploidy '$\psi$', a minor allele copy number '$M_i$', and a copy number per segment '$C_i$'.

The purity of a sample reflects the ratio of freely circulating DNA in a sample. E.g., the purity of a blood sample reflects the amount of tumor DNA in a blood sample.

The ploidy is the number of complete sets of chromosomes in a cell, and hence the number of possible alleles. Somatic cells in or from humans are normally diploid, carrying one set of 23 chromosomes from their father and one set of 23 chromosomes from their mother. In other word, the ploidy for normal human somatic cells is 2. As cancer cells are rapidly dividing, mistakes in the distribution of chromosomes can happen, resulting in some cells having too many chromosomes and others too few. Therefore, the cells in a tumor may have a different ploidy compared to healthy cells.

The minor allele copy number reflects the number of reads of the least frequent allele in a position. The copy number reflects the total number of allele reads. For example, when there are two alleles for a specific position, the copy number reflects the sum of the minor allele copy number and a major allele copy number.

Preferably, the estimating includes utilizing a gradient-descent method.

In particular, the purity is estimated using a gradient descent method, such as for example the Newton-Raphson method, to iteratively minimize a mean square error between old and new values for $\mathbb{E}[r_i]$ and $\mathbb{E}[m_i]$, where the log R ratios are '$r_i$' and the minor allele frequencies are '$m_i$' and the expectation value is based on a normal distribution fit.

This way, it can be avoided to perform the further steps of the evaluating method for a large number of purity values. Instead, the purity value can be reliably estimated, and the subsequent steps need to be performed only once. Therefore, the method is much faster and can easily be provided in a cloud-based setting.

Preferably, the first data represents allelic depths corresponding to a panel of less than 50 genes.

The present disclosure further relates to a method of determining a trend of a mutational burden, comprising a method as described above, wherein the first data represents allelic depths of a sample taken at a later time, and the second data represents allelic depths of a sample taken at a prior time.

The invention is further described with reference to the following figures:

Fig. 1 is a schematic overview of an exemplary method according to the present disclosure,
Fig. 2a shows a Haar wavelet (https://en.wikipedia.org/wiki/Haar_wavelet#/media/File:Haar_wavelet.svg),
Fig. 2b shows an exemplary Haar segmentation output,
Fig. 2c shows exemplary purity estimates and TMB counts per chromosome,
Fig. 2d shows Pareto Type I probability density functions with $x_m$ = 1 (https://en.wikipedia.org/wiki/File:Probability_density_function_of_Pareto_distribution.svg), and
Fig. 3 is an exemplary flow chart of an example according to the disclosure.

The method according to an exemplary method according to the present disclosure takes annotated Variant Call Format (VCF) files as inputs and parses them to generate log R ratios. The log R ratios are then used to segment the data. The segments are generated per chromosome by breaking them up into their orthonormal bases and clustering them based on roughly equal copy number. These segments are then used to estimate the purity, ploidy, minor allele copy number, and copy number per segment. The estimation is based on a parallelized gradient search which aims to minimize the mean squared error. These estimates are then used to compute the allele frequencies for both somatic and germline mutations per segment. These frequencies are subsequently used in a hypothesis test to classify a variant as somatic or germline. To eliminate uncertainties due to the threshold chosen for the hypothesis test, the classification is carried out by iterating over many thresholds. The total number of somatic mutations per iteration so obtained are then fit to a Pareto distribution. The mean (or the mean scaled by the standard deviation) of this fit is the Mutational Burden of a given sample.

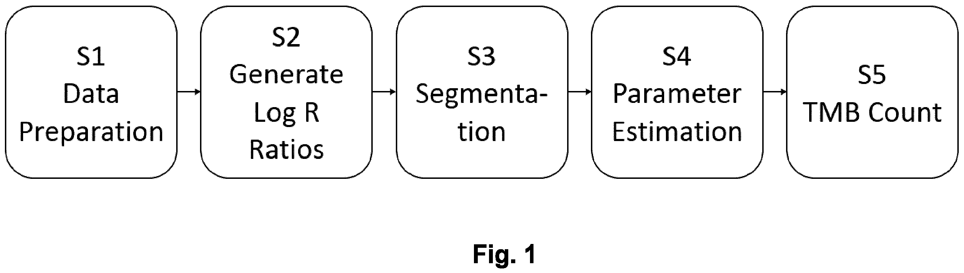

Fig. 1 schematically depicts an exemplary method according to the present disclosure. The method consists of five steps. The first step S1 is the 'Data Preparation' step wherein the VCF files are taken in. Depending on whether the user wants to use the method in 'two-sample' mode or 'one-sample' mode, one inputs the VCF file of the sample to be analyzed, i.e. the first data, along with the VCF of a matched sample or with the VCF of a process-matched normal sample, respectively. A process-matched normal sample is a 'typical' sample created using a library of normal samples (from healthy individuals) sequenced using the exact same procedure as that of the sample whose Mutational Burden is to be computed. If a sample-matched normal (as in the two-sample case) is missing, the process-matched normal is used in its place to eliminate process related errors.

The input VCF files are parsed to get the allelic depths from which 'log R ratios' are generated in S2. The log R ratios obtained are used in S3 (i.e., Segmentation) to break the genome/exome into smaller segments of roughly equal copy number. The segmentation is preferably carried out using the 'Haar Segmentation Algorithm', which is inherently parallelizable and is, therefore, fast and computationally efficient.

From the segments obtained above, to remove outliers, we filter segments based on the median segment size, i.e., all segments whose length is greater than or equal to one less than the median segment size are retained. The segments so obtained are then used in the 'Parameter Estimation' step to estimate the purity, ploidy, minor allele copy number, and the copy number per segment. These parameters are preferably estimated using the Newton-Raphson's method - a type of gradient-descent method - to iteratively minimize the mean squared error (MSE) between their new and old values. As with the previous step, this step is also easily parallelizable, thereby further reducing the computation time.

The allele frequencies for both somatic and germline mutations per segment are then computed using parameters estimated. In the final step S5 the mutations/variants are classified as somatic or germline using a standard 2-tailed binomial (allele frequency) based Hypothesis Test, where a given variant classification threshold is chosen. An intermediate Mutational Burden is then computed based on the aforementioned classification.

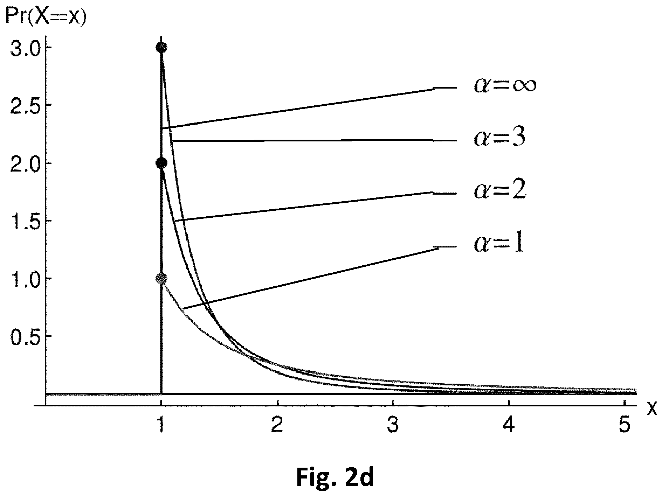

To eliminate the dependency on a classification threshold, which is widely prevalent in other algorithms, Mutational Burden values are computed for various classification thresholds. Since the Mutational Burden values are expected to saturate/stabilize after a given (sufficiently low) threshold, the (tumor) mutational burden (TMB) values obtained by the iterations are fit to a Pareto distribution, for example to a Pareto distribution similar to the ones depicted in Fig. 2d. This may thus serve as a hypothesis test using different thresholds. Finally, the mean (or the mean scaled by the standard deviation) of the Pareto fit is reported as the Mutational Burden value (in Mutations/megabase) of a given sample.

The method is now described in further detail. Therein, TMB refers to (tumor) mutational burden. The method needs to be able to compute TMB using a single sample while being panel and technology agnostic. In this disclosure, a method satisfying these criteria is described.

The method essentially consists of 5 steps which have been described above in conjunction with Fig. 1.

1. Initially, Fig. 1 shows a first step of Data Preparation (S1): The VCF file of the input sample is annotated and the VCF secondary analysis output is obtained. Then, the VCF files for the input sample as well as the process-matched normal sample are parsed.

In some embodiments, the input VCF file may be split per chromosome into smaller files for annotation. Merging is actually necessary only then. In a cloud based data annotation pipeline, the processing (or run) time is much smaller when the input VCF file is spilt per chromosome and processed in parallel.

2. Further, Fig. 1 shows a second step of Generating Log R Ratios (S2): The 'log R ratio' is the logarithm (to the base 2) of the ratio of the allelic depth of the mixed (input) sample to that of the process-matched normal sample for each position in the panel or genome/exome. These ratios are computed from the allelic depths obtained by parsing the VCF files in the step above.

3. Further, Fig. 1 shows a third step of Segmentation (S3): This is a process of breaking the genome/exome into smaller segments of roughly equal copy number. The method which is exemplary employed to carry this out is known as Haar Segmentation (Ben-Yaacov & Eldar, 2008) which can be summarised as follows:

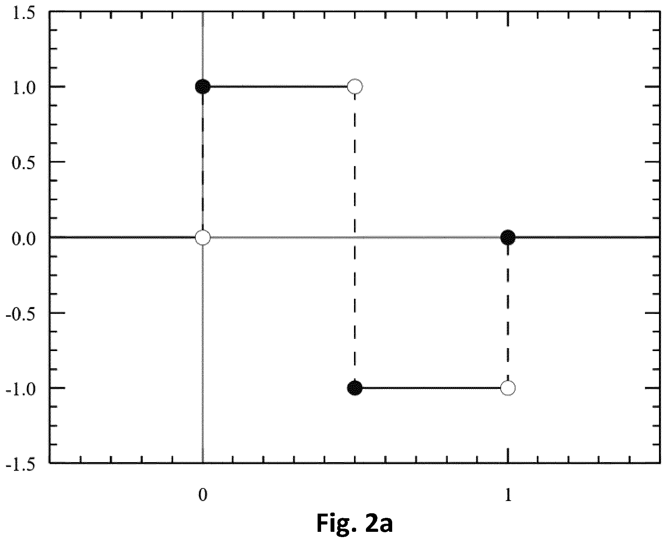

a. With log R ratios as the input, apply the undecimated (stationary) discrete wavelet transform (UDWT) using the Haar wavelet (cf. Figure 2a, taken from Wikipedia, "Haar Wavelet").
b. Select a set of subbands from the transform.
c. Find the local maxima of the selected subbands.
d. Threshold the maxima of each subband separately using the False Discovery Rate (FDR) thresholding procedure (Benjamini & Hochberg, 1995), where the FDR is defined as the proportion of false-positives out of all positives.
e. Unify the selected maxima from all the subbands to create a list of significant breakpoints in the data.
f. Reconstruct the segmentation result from the list of significant breakpoints.

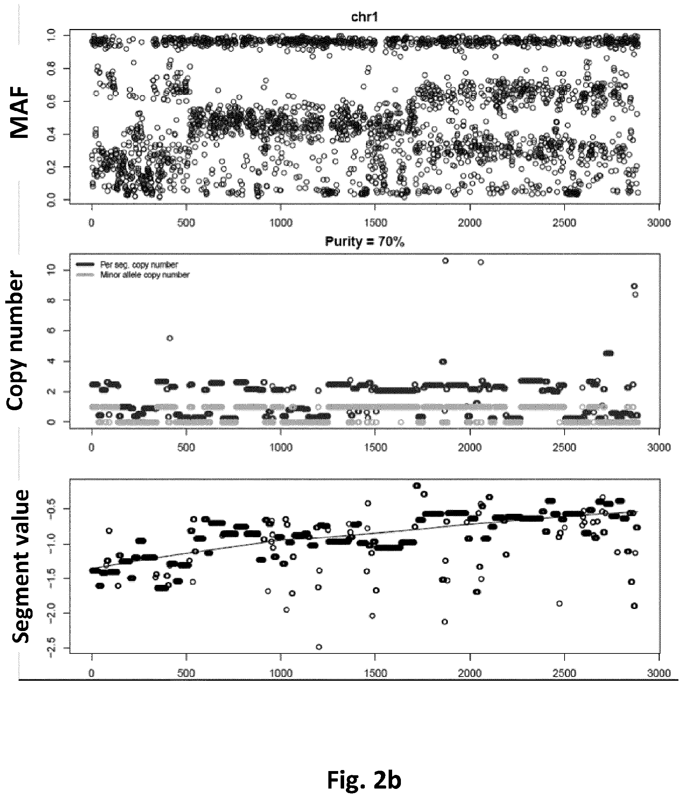

4. Further, Fig. 1 shows a fourth step of Parameter Estimation (S4): From the segments obtained using the method above (cf. Figure 2b, wherein MAF is the minor allele frequency), to remove outliers, segments are filtered based on the median segment size, i.e., all segments whose length is greater than or equal to one less than the median segment size are retained. Then, the parameters essential to determining the TMB are computed as described below.

a. For each segment '$i$' obtained using Haar segmentation, the log R ratios '$r_i$' and the minor allele frequencies '$m_i$' are fitted to a Normal distribution.
b. In a further sub-step, the means of the log R ratios as well as the minor allele frequencies per segment obtained by the fit are then described as

$$\mathbb{E}[r_i] = \log_2 \frac{pC_i + 2(1-p)}{p\Psi + 2(1-p)},$$

$$\mathbb{E}[m_i] = \frac{pM_i + 1 - p}{pC_i + 2(1-p)},$$

where 'p' is the purity, '$\psi$' is the ploidy, '$M_i$' is the minor allele copy number, and '$C_i$' is the copy number per segment.
c. As a starting point, the minor allele copy number is set to $M_i = C_i - 1$ and the initial values are computed for various purity values.

d. With the values computed based on such initial values, $\mathbb{E}[r_i]$ and $\mathbb{E}[m_i]$ are recomputed according to the provided formula. Subsequently, a mean squared error (MSE) between such computed new values and the formerly computed old values is calculated.
e. In a further sub-step, a gradient-descent method - preferably a Newton-Raphson method - is used to iteratively minimize the MSE (as computed in the step above).

e.i. In a further sub-step, an exponentially weighted average of the purity is computed across chromosomes. This way, the purity values do not vary significantly across chromosomes.

e.ii. The iterations using the gradient-descent method are continued so the minor allele frequency values get equal to those in the first data, for example in the original VCF file. Further, the iterations using the gradient-descent method are continued so the MSE between the log R ratios in the first data, for example in the original VCF file, and those that are computed is minimized. In other words, when a difference between the calculated minorallele frequency values and the minor allele frequency values according to the first data falls below a MAF threshold, and the MSE between the log R ratios in the first data the computed log R ratios falls below a MSE threshold, the iterations using the gradient-descent method are discontinued and the resulting parameters are used as estimated values.

f. In a further sub-step, the allele frequencies for both somatic ($f_{som}$) and germline ($f_{germ}$) mutations per segment are computed using the estimated values in the formulas

$$f_{germ} = \frac{p(C_i - M_i) + 1 - p}{pC_i + 2(1-p)},$$

and

$$f_{som} = \frac{p(C_i - M_i)}{pC_i + 2(1-p)}.$$

5. Further, Fig. 1 shows a fifth step of TMB Estimation (S5): In this case, computing the TMB entails classifying the mutations/variants as somatic or germline.

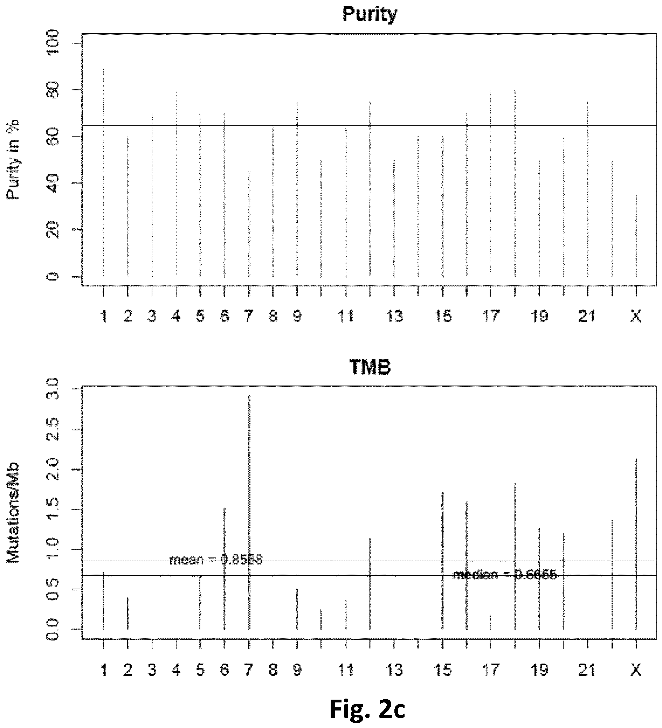

a. In a first sub-step, the desired classification is carried out using a standard 2-tailed binomial (allele frequency) based hypothesis test (cf. Figure 2c), where a given variant classification threshold '$\alpha$' for the p-value is chosen. The classification can be described as

$$classification = \begin{cases} germline, if\ (p - value_{som} \leq \alpha) \wedge (p - value_{germ} > \alpha) \\ somatic, if\ (p - value_{germ} \leq \alpha) \wedge (p - value_{som} > \alpha) \end{cases}.$$

b. In a further sub-step after the classifying, the number of somatic mutations in the tumor sample is multiplied by

$10^6$ and divided by its respective exon/panel length. The resulting value is an intermediate value for a TMB whose units are mutations/megabase.

c.i. The classification using a hypothesis test and the calculation of an intermediate value is repeated for various classification thresholds, where the value of $\alpha$ at a given iteration is lower than the previous iteration. E.g., for iteration

$$\alpha_i = \frac{\alpha_{i-1}}{100}$$

i, . (Note: the number of iterations is inversely proportional to the sample purity, viz. lower the purity, larger the number of iterations that need to be carried out.)

This way, a dependency of the intermediate value from the classification threshold '$\alpha$' and the purity of the sample is mitigated. The intermediate value depends on the purity as the probability of correct classification of somatic and germline mutations depends on (and is directly proportional to) the purity of the sample. Therefore, by calculating multiple intermediate values, i.e. by performing multiple iterations of the prior sub-steps, it is ensured that a stable TMB value is obtained.

c.ii. Therein, the total number of iterations '$n$' carried out can be selected in one of two ways:

c.ii.1. A fixed number of iterations depending on the purity of the sample is selected. For example, $n = 50$ may be selected for purity between 50 - 70%. Alternatively, $n = 100$ may be selected for a purity between 50-70%. As another example, $n = 100$ may be selected for purity < 30%. Alternatively, $n = 200$ may be selected for a purity < 30%.

c.ii.2. Alternatively, the number of iterations can be chosen depending on the meta information of the samples (if available), i.e. the total number of samples whose TMB values need to be computed, whether or not they belong to the same cancer type, or similar meta information. For example, if there are more than 5 samples of a given cancer type, the range of values '$n$' takes can be chosen based on the average purity of the set of samples and the standard deviation of the purity within the sample set.

c.iii. The TMB values obtained by the iterations above are then fitted to a heavy tail distribution, for example to a Pareto distribution (cf. Figure 2d, source: Wikipedia, "Pareto Distribution") whose parameters are computed as follows:

c.iii.1. For TMB values $X = \{X1, \cdots, Xn\}$ resulting from the iterations in step 5.c.i., the lower bound of the support for

$$\widehat{m} = \min_i X_i$$

the Pareto distribution is determined as , where $i \in \{1, \cdots, n\}$.

c.iii.2. The Maximum Likelihood Estimate (MLE) of the Pareto scaling parameter $\hat{\beta}$ is determined as

$$\hat{\beta} = \frac{n}{\sum_{i=1}^{n} ln \frac{X_i}{\widehat{m}}}$$

c.iii.3. Using $\hat{m}$ and $\hat{\beta}$, the Pareto distribution describing the TMB values for a given sample is characterized by:

$$\text{c.iii.3.a. Mean, } \mathbb{E}[X] = \begin{cases} \infty, for \hat{\beta} \leq 1 \\ \frac{\widehat{m}\hat{\beta}}{(\hat{\beta}-1)}, for \hat{\beta} > 1 \end{cases}$$

$$\text{c.iii.3.b. Variance, } Var[X] = \begin{cases} \infty, \text{ for } \widehat{\beta} \leq 2 \\ \frac{(\widehat{m})^2\widehat{\beta}}{(\hat{\beta}-1)^2(\hat{\beta}-2)}, \text{for } \hat{\beta} > 2 \end{cases}$$

c.iii.4. The final TMB value (in Mutations/megabase) for a given sample 'X' is then given as

$$TMB_X = \begin{cases} \dfrac{\mathbb{E}[X]}{\sqrt{Var[X]}}, \text{for } Var[X] \neq \infty \\ \mathbb{E}[X], \text{for } Var[X] = \infty \end{cases}$$

c.iii.4.a. If the process-matched normal sample has been replaced by a tumor sample from a different point in time, the final TMB value (in Mutations/megabase) for a given sample 'X' is given as $\text{TMB}_X = \mathbb{E}[X]$ .

The intermediate values, which represent TMB value estimates, saturate and stabilize after a sufficiently low threshold. Therefore, using the fitting to a heavy tail distribution above, a reliable and robust final estimation for the TMB is obtained very fast.

Several aspects of embodiments according to Fig. 1 are further illustrated in Fig. 2a to Fig. 2d.

Fig. 2a shows an exemplary wavelet, namely the Haar wavelet. The Haar wavelet is composed as a concatenation of two rectangular functions and is -1 for values below zero and one above zero. Based on the Haar wavelet, the fast and efficient Haar transform may be applied to the rate data. The Haar transform is a continuous signal which results from applying a shifted Haar wavelet to each of a discrete input values and summarizing the result.

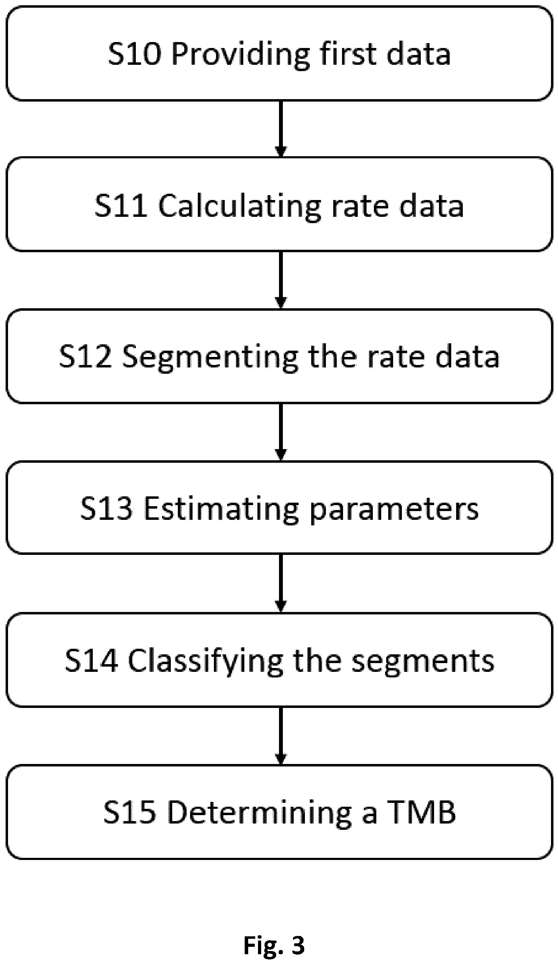

Fig. 2b shows an exemplary Haar segmentation output. Fig. 2b may show an exemplary input and result of step S3 according to embodiments as described herein with reference to Fig. 1. Further, Fig. 2b may show an exemplary input and result of step S12 according to embodiments as will be described herein with reference to Fig. 3. Fig. 2b includes three diagrams. The first diagram represents the MAF, that is the minor allele frequency, for each gene location. The second diagram represents the copy number per gene location and the minor allele copy number, which is a subset of the total copy number, per gene location. The third diagram represents the segment value, which is calculated using step S3 or S12 from the MAF and the copy numbers as input data. As is illustrated in the third diagram, segments of contiguous gene locations are easily identifiable, and outliers are recognizable.

Fig. 2c shows exemplary purity estimates and TMB counts per chromosome. Fig. 2c includes two diagrams, wherein the first diagram represents exemplary purity estimates and the second diagram represents exemplary TMB counts per chromosome. The purity estimates and/or the TMB counts per chromosome show an exemplary result of steps S4 and S5 according to embodiments as described herein with reference to Fig. 1, based on exemplary data. Further, the purity estimates and/or the TMB counts per chromosome may be exemplary results of steps S13 to S15 according to embodiments as will be described herein with reference to Fig. 3.

The first diagram in Fig. 2c shows, in a vertical dimension, a purity value for each of a number of chromosomes in a horizontal dimension. Thus, there is one line representing the purity for each chromosome. The purity values may range from 0% purity to 100% purity. Further, the first diagram in Fig. 2c shows a mean purity as a horizontal line at a level of about 65% purity. The mean purity is a mean taken across the purity values for all chromosomes.

The second diagram in Fig. 2c shows, in a vertical dimension, a number of mutations per megabase for each of a number of chromosomes in a horizontal dimension. Thus, there is one line representing the TMB for each chromosome. The TMB values range from 0.0 to 3.0 in the shown exemplary case. Further, the second diagram in Fig. 2c shows a mean TMB as a horizontal line at a level of 0.8568, and a median TMB as a horizontal line at a level of 0.6655. The mean TMB is a mean taken across the TMB values for all chromosomes. The median TMB is a value chosen such that the TMB for one half of the chromosomes lies below the median TMB, and the TMB for another half of the chromosomes lies above the median TMB.

Fig. 2d shows exemplary Pareto distributions which may be used in step c.iii as described above. The exemplary Pareto distribution may be used in step S5 of an embodiment according to Fig. 1 as described herein. Further, the exemplary Pareto distribution may be used in step S14 of an embodiment according to Fig. 3 as described herein. More precisely, Fig. 2d shows four Pareto Type I probability density functions. The probability density function of a Pareto Type I distribution is given by the equations $f(x) = \dfrac{\alpha x_{min}^{\alpha}}{x^{\alpha+1}}$ for $x \geq x_{min}$ and $f(x) = 0$ for $x < x_{min}$.

Here $x_{min}$ is a parameter that describes the minimum value of the distribution. This is also the mode of the distribution, i.e. the maximum value of the probability density. As the distance between $x$ and $x_{min}$ increases, the probability that $X$ takes the value $x$ decreases. The distance between the two values is determined as the quotient, that is, the ratio between the two quantities. Thus, the Pareto distribution is a heavy-tail distribution.

The parameter $\alpha$ describes the size ratio of the random values depending on their frequency. With $\alpha$ the quotient is exponentiated. With a larger $\alpha$, the curve is significantly steeper, which means that the random variable $X$ takes on large values with lower probability.

Fig. 2d shows four such Pareto Type I probability density functions in a single graph, wherein $x_{min}$ = 1 for each of them. The example where $\alpha = \infty$ results in the straight vertical ray at $x$ = 1 and the straight horizontal ray at $y$ = 0. The three curves show the probability density functions for the example where $\alpha$ = 3, $\alpha$ = 2, and $\alpha$ = 1, respectively.

As has been described, Fig. 2a to Fig. 2d illustrate several aspects of embodiments according to Fig. 1 and of embodiments according to Fig. 3.

In other words, and with reference to Fig. 3 showing a basic flow chart of an example, the present disclosure relates to a method of evaluating a (tumor) mutational burden in a sample, e.g. a liquid biopsy sample. The method comprises providing S10 first data which represents allelic depths of the sample and calculating S11 rate data based on the first data, segmenting S12 the rate data into a plurality of segments of rate data using a wavelet transform. Preferably, the plurality of segments is filtered and fitted to a distribution. According to an example as described above, the wavelet transform is used to get the segments, filter the segments based on median size, and the fit the log R ratios and the minor allele frequencies to a Normal distribution.

The method further comprises estimating S13 parameters for each of the plurality of segments, preferably based on the first data. Then, each of the (filtered) plurality of segments is classified S14 based on the estimated parameters, wherein the classifying includes, for each of a plurality of classification thresholds, generating a classification for the (filtered) plurality of segments based on the classification threshold and determining an intermediate value, i.e. mutational burden value, based on the classification. A mutational burden (TMB) of the sample is determined S15 based on the intermediate values.

The segmenting may include filtering and fitting the plurality of segments to a distribution. Alternatively or additionally, the determining may include fitting the intermediate values to a heavy-tail distribution, e.g. a Pareto distribution, calculating one of a mean or a mean scaled by the standard deviation of the fitted heavy-tail distribution, and providing the calculated value as the mutational burden.

Furthermore, second data may be provided which represents allelic depths which may be sample-matched or process-matched, wherein the rate data is based on the first data and the second data. The segmenting may comprise utilizing a Haar wavelet transform, for example using a Haar wavelet as depicted in Fig. 2a.

The estimated parameters may include at least one of purity, ploidy, minor allele copy number, and copy number per segment. The estimating may include utilizing a gradient-descent method, preferably a Newton-Raphson method.

The method according to present disclosure does not require first data representing allelic depths corresponding to a large panel. It can also be used with a small panel. In particular, the method according to present disclosure can be used with smaller panels than other methods. The first data preferably may represent allelic depths corresponding to a panel of less than 50 genes, more preferred a panel of less than 20 genes.

The present disclosure further relates to a method of determining a trend of a mutational burden comprising a method as described herein. The first data represents allelic depths of a sample taken at a later time, and the second data represents allelic depths of a sample taken at a prior time. The trend or variation of a mutational burden which can be determined according to this method may be in reaction to a treatment or due to the patient's inherent immune response, the diet, etc. Trend may thus be understood as variation, aberrance, anomaly or deviation from prior or expected values.

The disclosure may also comprise a data processing apparatus configured to perform the above method. Certain steps or all steps may be performed locally or cloud-based.

According to the present disclosure, a method is provided which is capable of finding (or computing) the Mutational Burden (or Load) as well as sample purity for both solid tumor and liquid biopsy samples in a fast and efficient manner for both Whole Exome Sequencing as well as panel-based assays.

The method may be implemented as a cloud-based pipeline and requires lower coverage than the prior art (on average), >100x versus >500x. Furthermore, UMIs (Unique Molecular Identifiers) are not needed. A proprietary and a non-proprietary pipeline can be used. As one example, a pipeline based on the Broad Institute recommendation (GATK, Genome Analysis Tool Kit) can be used. A proprietary pipeline may be advantageous, for example it may result in a faster or more reliable method. The purity of the sample can be estimated and no user input is required.

Other aspects, features, and advantages will be apparent from the summary above, as well as from the description that follows, including the figures and the claims.

While the invention has been illustrated and described in detail in the drawings and foregoing description, such illustration and description are to be considered illustrative or exemplary and not restrictive. It will be understood that changes and modifications may be made by those of ordinary skill within the scope of the following claims. In particular, the present invention covers further embodiments with any combination of features from different embodiments described above and below.

Furthermore, in the claims the word "comprising" does not exclude other elements or steps, and the indefinite article "a" or "an" does not exclude a plurality. A single unit may fulfil the functions of several features recited in the claims. The terms "essentially", "about", "approximately" and the like in connection with an attribute or a value particularly also define exactly the attribute or exactly the value, respectively. Any reference signs in the claims should not be construed as limiting the scope.

**Claims**

1. Method of evaluating a mutational burden in a sample, said method including:

   Providing (S10) first data which represents allelic depths of the sample;
   calculating (S11) rate data based on the first data;
   segmenting (S12) the rate data into a plurality of segments of rate data using a wavelet transform;
   estimating (S13) parameters for each of the plurality of segments;
   classifying (S14) each of the plurality of segments based on the estimated parameters;
   wherein the classifying includes, for each of a plurality of classification thresholds, generating a classification for the plurality of segments based on the classification threshold and determining an intermediate value based on the classification;
   determining (S15) a mutational burden of the sample based on the intermediate values.

2. The method of claim 1, wherein the segmenting includes filtering and fitting the plurality of segments to a distribution, and/or

   wherein the determining includes fitting the intermediate values to a heavy-tail distribution; calculating one of a mean or a mean scaled by the standard deviation of the fitted heavy-tail distribution;
   and providing the calculated value as the mutational burden.

3. The method of claim 1 or 2, further comprising providing second data which represents allelic depths, wherein the rate data is based on the first data and the second data.

4. The method of one of claims 1 to 3, wherein the segmenting comprises utilizing a Haar wavelet transform.

5. The method of claim 4, wherein the estimated parameters include at least one of purity, ploidy, minor allele copy number, and copy number per segment.

6. The method of any one of claims 4 or 5, wherein the estimating includes utilizing a gradient-descent method.

7. The method of any one of claims 1 to 6, wherein the first data represents allelic depths corresponding to a panel of less than 50 genes, preferably less than 20 genes.

8. Method of determining a trend of a mutational burden, comprising a method according to any one of claims 3 to 7, wherein the first data represents allelic depths of a sample taken at a later time, and the second data represents allelic depths of a sample taken at a prior time.

9. Use of a method according to one of claims 1 to 8 to evaluate in tumor mutational burden in a liquid biopsy sample.

**Fig. 1**

EP 4 123 653 A1

**Fig. 2a**

**Fig. 2b**

## Purity

## TMB

**Fig. 2c**

15

**Fig. 2d**

S10 Providing first data

S11 Calculating rate data

S12 Segmenting the rate data

S13 Estimating parameters

S14 Classifying the segments

S15 Determining a TMB

**Fig. 3**

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | JAMES X. SUN ET AL: "A computational approach to distinguish somatic vs. germline origin of genomic alterations from deep sequencing of cancer specimens without a matched normal", PLOS COMPUTATIONAL BIOLOGY, vol. 14, no. 2, 7 February 2018 (2018-02-07), page e1005965, XP055586692, DOI: 10.1371/journal.pcbi.1005965 * whole document, in particular abstract, first paragraph of Introduction on page 2; Methods; page 10, last 5 lines; Table 1 and supplementary material. * | 1-9 | INV. G16B20/10 G16B20/20 |
| X,D | US 2018/363066 A1 (CHALMERS ZACHARY R [US] ET AL) 20 December 2018 (2018-12-20) * whole document, in particular claims * | 1-9 | |
| A | MELENDEZ ET AL.: "Methods of measurement for tumor mutational burden in tumor tissue", TRANSLATIONAL LUNG CANCER RESEARCH, vol. 7, no. 5, 1 December 2018 (2018-12-01), pages 661-667, XP055787276, Hong Kong ISSN: 2218-6751, DOI: 10.21037/tlcr.2018.08.02 * the whole document * | 1-9 | TECHNICAL FIELDS SEARCHED (IPC)<br><br>G16B |

-/--

The present search report has been drawn up for all claims

| Place of search | Date of completion of the search | Examiner |
|---|---|---|
| Munich | 11 January 2022 | Vanmontfort, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 21 18 7249

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | STENZINGER ALBRECHT ET AL: "Harmonization and Standardization of Panel-Based Tumor Mutational Burden Measurement: Real-World Results and Recommendations of the Quality in Pathology Study", JOURNAL OF THORACIC ONCOLOGY, vol. 15, no. 7, 1 July 2020 (2020-07-01), pages 1177-1189, XP055877082, US ISSN: 1556-0864, DOI: 10.1016/j.jtho.2020.01.023 * whole document, in particular abstract; Figures and Discussion * | 1-9 | |

-----

TECHNICAL FIELDS
SEARCHED     (IPC)

The present search report has been drawn up for all claims

| Place of search | Date of completion of the search | Examiner |
|---|---|---|
| Munich | 11 January 2022 | Vanmontfort, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EP 4 123 653 A1

## ANNEX TO THE EUROPEAN SEARCH REPORT
## ON EUROPEAN PATENT APPLICATION NO.

EP 21 18 7249

11-01-2022

| Patent document cited in search report | Publication date | Patent family member(s) | Publication date |
|---|---|---|---|
| US 2018363066 A1 | 20-12-2018 | AU 2017225876 A1 | 30-08-2018 |
| | | AU 2021203640 A1 | 01-07-2021 |
| | | CA 3014653 A1 | 08-09-2017 |
| | | CN 109196359 A | 11-01-2019 |
| | | EP 3423828 A1 | 09-01-2019 |
| | | JP 6930992 B2 | 01-09-2021 |
| | | JP 2019512218 A | 16-05-2019 |
| | | JP 2021191276 A | 16-12-2021 |
| | | KR 20180130506 A | 07-12-2018 |
| | | US 2018363066 A1 | 20-12-2018 |
| | | WO 2017151524 A1 | 08-09-2017 |

**EP 4 123 653 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9792403 B2
- US 20180363066 A1

**Non-patent literature cited in the description**

- **SUN JX et al.** *PLOS Computational Biology,* February 2018, vol. 14, 1-13
- **PETER VAN LOO et al.** *Proceedings of the National Academy of Sciences,* 2010, vol. 107 (39), 16910-16915